# EUROPEAN PATENT SPECIFICATION

(11) **EP 1 933 146 B1**
(45) Date of publication and mention of the grant of the patent: **24.08.2011**
(21) Application number: 06025529.6
(22) Date of filing: 11.12.2006
(51) Int. Cl.: G01N 33/558, G01N 33/543, G01N 33/58, C12Q 1/68

(54) **Rapid immunochromatographic detection by amplification of the colloidal gold signal**
Schnelle immunchromatographische Detektion durch Verstärkung des kolloidalen Gold Signals
Détection rapide immunochromatographique par amplification du signal d'or colloïdal

(43) Date of publication of application: 18.06.2008
(73) Proprietor: AraGen Biotechnology Co. Ltd., 11710 Naor (JO)
(72) Inventor: Badwan, Adnan, Dr., 11185 Amman (JO); Murshed, Abedel-qader Mohammed, 11118 Amman (JO)
(74) Representative: Winkler, Andreas Fritz Ernst

(56) References cited:
- EP-A- 0 590 695
- WO-A-02/13685
- GB-A- 2 284 479
- US-A1- 2005 164 405
- WANG ET AL: "Development of an immunochromatographic test to detect White Spot Syndrome Virus of shrimp" AQUACULTURE, vol. 255, no. 1-4, 31 May 2006 (2006-05-31), pages 196-200, XP005464138
- OH J ET AL: "Evaluation of GenediaR HBsAg Rapid and GenediaR Anti-HBs Rapid for the Screening of HBsAg and Anti-HBs" KOREAN J CLIN PATHOL, vol. 19, 1999, pages 114-117, XP002424939

## Description

The present invention relates in general to the field of diagnostics, namely to a device for the detection of a target in a sample. More precisely, the present invention relates to a rapid immunochromatographic test device especially suitable for ultra-sensitivity detection of an antibody and/or antigen in a sample using double sandwich immunoassay detection for sensitivity enhancement by signal amplification. The present invention further refers to a method for the production of the test device, to the uses of the test device for the early detection of disease infection such as HIV in a sample, as well as to a kit comprising the test device.

### BACKGROUND OF THE INVENTION

In recent years the *in vitro* diagnostics (IVD) industry has made enormous efforts to develop immunochromatographic tests. Such tests have found applications in both clinical and non-clinical fields¹. A clinical utility of this test format has been shown for more than 150 different analytes, and many of them are target now of commercially available diagnostic products³. The wide range of applications for such devices has been reviewed^{1,2}.

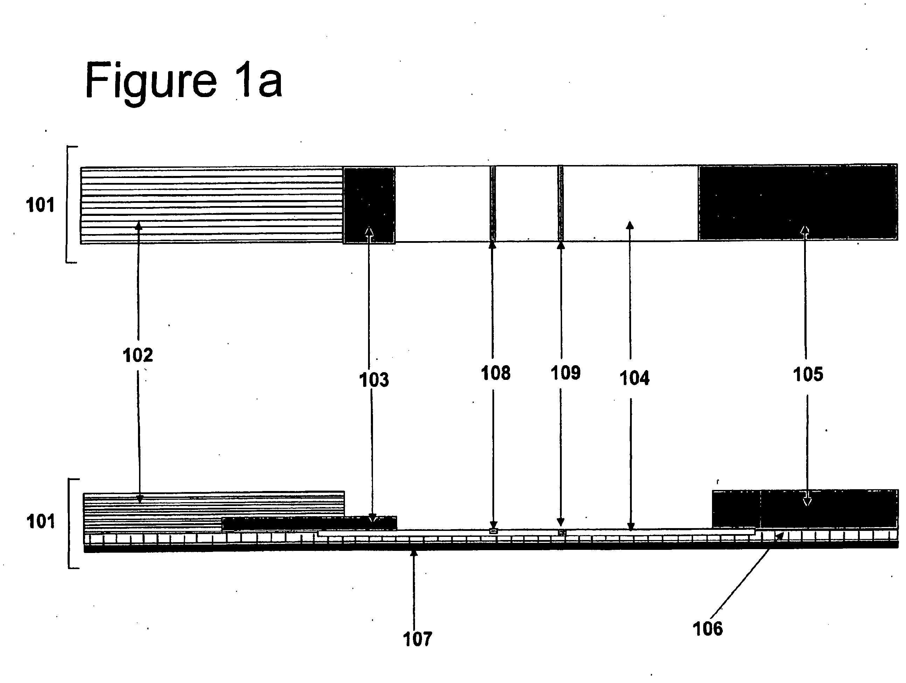

Rapid immunochromatographic test devices, e.g. in the form of a test strip, are made up of a number of components (Figure 1a). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zone) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

However, despite the wide use of rapid immunochromatographic test devices, their suitability is still limited with regard to certain applications. Urine, for example, contains very low levels of IgG, frequently around 1 mg/l. Therefore, the detection of antibodies, e.g. directed to HIV or HCV, require very sensitive techniques. To date, the tests for antibodies in urine samples are based on ELISA and Western blot techniques, which are labour-intensive, time-consuming and need to be carried out by qualified persons. Efforts are being made to develop simple and/or rapid tests for the detection of antibody to HIV in urine specimens ⁴.

Oral fluid specimens consist often of saliva, which predominantly contains IgA class antibody, and oral mucosal transudates, which mostly contain IgG, and therefore also have much lower levels of IgG than serum. The levels of IgG normally found in oral fluid specimens (approximately 15 mg/l) are, however, higher than in urine specimens and innovative simple and rapid technology that has been shown to be effective for whole blood, serum and plasma, e.g. lateral flow through a chromatographic membrane, has been developed for use with these specimens ⁴.

Human chorionic gonadotropin (hCG) is a glycopeptide hormone produced by the placenta during pregnancy. The appearance and rapid increase in the concentration of hCG in the subject's urine makes it a good marker for confirming pregnancy. The concentration of hCG in urine increases steadily to a circulation peak of as much as 50,000 mIU/ml between the eighth and eleventh weeks.
Urine hCG levels during pregnancy are estimated to be:
1. 10-30 mIU/ml 7-10 days post conception.
2. 37,000-50,000 mIU/ml 8-11 weeks after last menstrual period.
3. <5 mIU/ml Healthy men or non-pregnant women.
In the prior art the hCG test is a chromatographic immunoassay which uses specific antibodies to selectively identify hCG in urine with a high degree of sensitivity. Elevated levels of hCG as low as 20 mIU/ml can be detected within 3 minutes.

So far there are several tests used to detect the presence of hepatitis B antibodies. In addition, there are also several tests in the prior art that detect the presence of viral antigens.

The hepatitis B surface antibody (anti-HBs) detection is one of the most common test. Its presence indicates previous exposure to HBV, but the virus is no longer present and the person cannot pass on the virus to others. The antibody also protects the body from future HBV infection. In addition to exposure to HBV, the antibodies can also be acquired from successful vaccination. This test is done to determine the need for vaccination (if anti-HBs is absent), or following the completion of vaccination against the disease, or following an active infection. Hepatitis B surface antigen (HBsAg) is a protein antigen produced by HBV. This antigen is the earliest indicator of acute hepatitis B and frequently identifies infected people before symptoms appear. HBsAg disappears from the blood during the recovery period. In some people (particularly those infected as children or those with a weak immune system, such as those with AIDS), chronic infection with HBV may occur and HBsAg remains positive.

Further, testing for HIV is an essential component in the diagnosis and treatment of persons infected with the virus, in screening of blood for transfusion, in surveillance and in HIV/AIDS related research. Thus accurate and cost-effective testing is of great importance in combating the spread of HIV. It is imperative that tests for the diagnosis of HIV infection be as accurate as possible, given the serious ethical, legal and social issues that accompany HIV infection.

The number of people living with HIV has now risen to reach its highest level ever: close to 40 million people are living with the virus and close to 5 million people were newly infected with HIV in 2004 alone. Worldwide, the AIDS epidemic killed over 3 million people last year alone (Source: UNAIDS). Furthermore, only one in five people needing HIV prevention worldwide have access to basic prevention services and only one in ten people living with HIV has been tested for the virus.

The HI virus is most easily transmitted to others during the initial period of acute HIV infection, when the viral load (quantity of HIV RNA in the blood) is especially high and when people are not aware of being contaminated by the virus. Most HIV infections are transmitted at this stage, called primary infection. Earlier detection using ultra sensitive tests avoids missing primary infections, enabling immediate precautionary measures to be taken to help prevent the risk of HIV transmission to a non-infected partner, to an unborn child, or through blood donations or direct blood contact. Earlier detection of HIV infection also ensures the implementation of early antiretroviral therapy (ART) to slow down the progression of HIV infection, thereby improving patient care and quality of life.

The diagnosis of HIV infection is usually made on the basis of the detection of HIV antibodies and/or antigen. The diagnosis of an HIV infection can be made indirectly, i.e. through the demonstration of virus-specific antibodies. Besides such indirect diagnosis based on detection of antibodies, a direct diagnosis of HIV infection is also possible: either through the demonstration of infectious virus (using cell culture), viral antigens (p24 antigen ELISA) or viral nucleic acid (i.e. viral genome); the latter is also termed nucleic acid testing (NAT).

One important problem of HIV antibody testing is the so-called "diagnostic window". This is the time period that elapses between the time of acquisition of HIV infection until detectable levels of antibodies are present. The switch from antibody-negative to antibody-positive is called "seroconversion".

The most widely used screening tests are ELISAs as they are the most appropriate for screening large numbers of specimens on a daily basis, e.g. blood donations. The earliest assays used purified HIV lysates (1st generation assays). Improved assays based on recombinant proteins and/or synthetic peptides, which also enabled the production of combined HIV-1/HIV-2 assays, became rapidly available (2nd generation assays). The so-called 3rd generation or antigen-sandwich assays, which use labelled antigens as conjugate, are more sensitive and have reduced the diagnostic window period considerably^{5,6}.

WO 02/13685 refers to a pregnancy diagnosis device for distinguishing between a normal pregnancy and an ectopic pregnancy by immunologically detecting morphological differences between hCG and modified forms thereof.

US 2005/164405 refers to a chromatographic immunoassay test strip in which an analyte in a sample reacts with a ligand to form a "ligand A-analyte-ligand B/tracer" first, then is captured by a bridge immobilized on the test zone of a solid phase to form a complex of "bridge-ligand A-analyte-ligand B/tracer" which can then be detected.

EP 0 590 695 refers to a liquid transfer device for use in assay procedures comprising a sheet of porous material for capillary liquid flow therethrough.

GB 2 284 479 refers to a liquid transfer device having utility in diagnostic assays comprising first and second capillary flow channels.

Wang et al (2006) (20) refers to an immunochromatographic test device for detecting white spot syndrome virus of shrimp using monoclonal antibodies labeled with colloidal gold as a detection reagent and a capture antibody immobilized on a nitrocellulose membrane.

Oh et al. (1999) (21) refers to a rapid screening kit for the detection of hepatitis surface antigen and antibody using an immunochromatographic method.

Thus, there is need in the prior art to provide a rapid immunochromatographic test device suitable for the ultra-sensitive detection of target in a sample.

It is an object of the present invention to overcome the problems especially with regard to the applicability of rapid immunochromatographic test devices for the detection of hCG, HBsAG, anti-HBs, IgG, e.g. HIV antibodies, in urine, blood, serum or saliva by enhanced sensitivity.

It is therefore an object to enhance the sensitivity of the rapid immunochromatographic detection system. Thus, it is an object of the present invention to overcome the drawbacks of the prior art and to provide especially a simple and rapid test device for the ultra-sensitive antibody and/or antigen detection by signal development and signal amplification suitable to be employed for the early detection of disease infections in a sample.

### SUMMARY OF THE INVENTION

In one embodiment the present invention concerns a rapid immunochromatographic test device for the detection of a target in a sample, comprising
(a) a first gold conjugate releasing pad, comprising colloidal gold conjugated with a first specific antibody or specific antigen to capture the target from a first site,
(b) a second gold conjugate releasing pad, comprising colloidal gold conjugated with a second specific antibody or specific antigen to capture the target from a second site; and
(c) a capture test zone comprising the second specific antibody or specific antigen; wherein both releasing pads are located at different positions within the test device such that the target in the sample will be captured by the first specific antibody or specific antigen conjugated to the first colloidal gold to form a complex "target-first colloidal gold conjugate", the complex will be captured then by the second specific antibody or specific antigen and kept within the test zone, and then the second colloidal gold conjugated with the second specific antibody or specific antigen will be released to capture the target.

In a further embodiment the present invention concerns a method for the production of a device according to the present invention comprising the steps of
a. preparing a colloidal gold solution;
b. preparing a conjugation buffer;
c. partitioning the conjugation buffer by dividing it into a first and a second flask;
d. adding a first specific antibody according to claim 9 to the conjugation buffer in the first flask;
e. adding a second specific antibody according to claim 9 to the conjugation buffer in the second flask, wherein said antibody differs from the antibody used in step d) in that the two antibodies capture the target from two different sites;
f. adding colloidal gold solution into each flask;
g. adding stabilizing buffer to each flask;
h. concentrating each conjugate;
i. adding a surfactant to the first conjugate and soaking glass fibre sheet conjugate pad into the conjugate;
j. soaking another glass fibre sheet conjugate pad into the second conjugate;
k. printing capturing/sample and control lines onto the membrane (104), wherein the sample line comprises the second specific antibody;
l. laminating cards using the glass fibre sheet conjugate pad comprising the first gold conjugate; and
m. cutting cards into strips.

In another embodiment the present invention relates to the use of a device according to the present invention for the detection of a disease in at least one sample.

In a further embodiment the present invention refers to a kit for detection of a disease comprising the device according to the present invention and a manual.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

As outlined above there is a need in the prior art to provide a new test device suitable for the early detection of a disease infection in at least one sample. Further, there is a need in the prior art to provide a new method for rapid immunochromatographic detection of a target in a sample for the detection of a disease or a specific condition such as pregnancy in a subject. In addition, there is also a need in the art for devices suitable for simple, rapid and ultra-sensitive detection of an antigen and/or antibody, which devices having a higher sensitivity than devices from the prior art.

In a first aspect the present invention provides a rapid immunochromatographic test device for the detection of a target in a sample, comprising
(a) a first gold conjugate releasing pad, comprising colloidal gold conjugated with a first specific antibody or specific antigen to capture the target from a first site,
(b) a second gold conjugate releasing pad, comprising colloidal gold conjugated with a second specific antibody or specific antigen to capture the target from a second site; and
(c) a capture test zone comprising the second specific antibody or specific antigen; wherein both releasing pads are located at different positions within the test device such that the target in the sample will be captured by the first specific antibody or specific antigen conjugated to the first colloidal gold to form a complex "target-first colloidal gold conjugate", the complex will be captured then by the second specific antibody or specific antigen and kept within the test zone, and then the second colloidal gold conjugated with the second specific antibody or specific antigen will be released to capture the target.
wherein both releasing pads are located at different positions within the test device.

The first colloidal gold conjugated with a first antibody or antigen captures the target in the sample and forms a complex "target-first colloidal conjugate". Preferably this target in the sample is an antigen and/or antibody.

In the device according to the present invention the first gold conjugate releasing pad comprises a gold conjugate that is conjugated with a first specific antibody or antigen to capture the target analyte from the first site. The second gold conjugate releasing pad comprises a gold conjugated with a second specific antibody or antigen to capture the target analyte from the second site. The last mentioned conjugated antibody or antigen is the same antibody or antigen that is immobilized onto the nitrocellulose membrane.

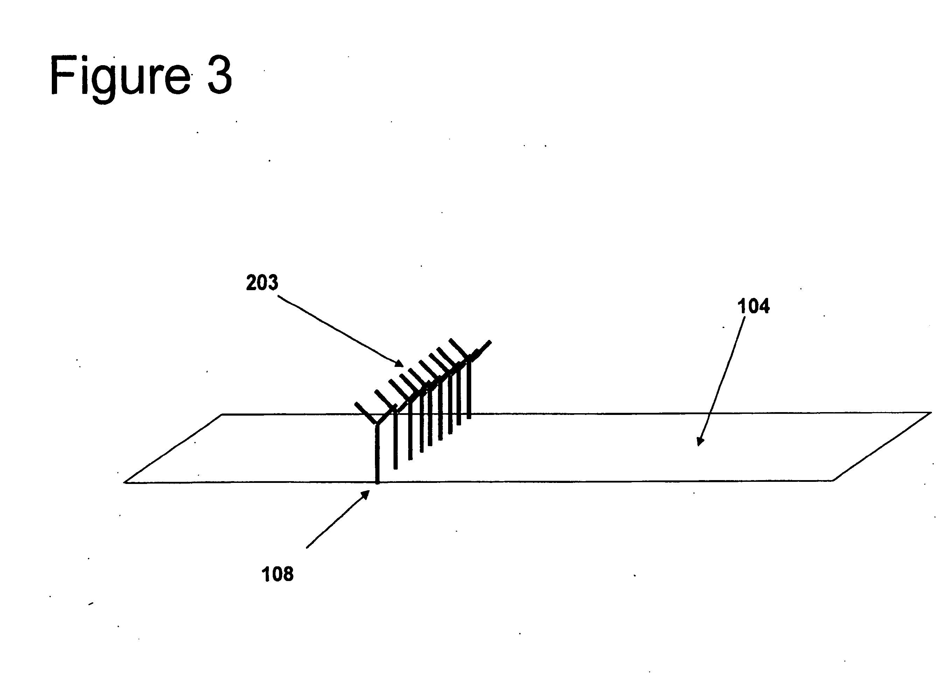

In one preferred embodiment of the device according to the present invention the first gold conjugate releasing pad comprises a gold conjugate 201 that is conjugated with a first specific antibody 202 or antigen to capture the target analyte from the first site 202'. The second gold conjugate releasing pad comprises a gold 211 conjugated with a second specific antibody 203 or antigen to capture the target analyte from the second site 203'. The last mentioned conjugated antibody 203 or antigen is the same antibody or antigen that is immobilized onto the nitrocellulose membrane (Figure 3).

In another embodiment of the device according to the present invention the device further comprises a test strip comprising
a) a sample pad,
b) a conjugate pad comprising the first gold conjugate releasing pad,
c) a conjugate pad comprising the second gold conjugate releasing pad,
d) a membrane comprising a capture test zone and a negative control zone, and
e) an absorbent pad.

In the device according to the present invention the capture test zone comprises the second antibody or antigen. The antibody or antigen within the test zone capture the target from a site that differs from that site captured by the first antibody conjugated with the first colloidal gold, why both antibodies differ from each other.

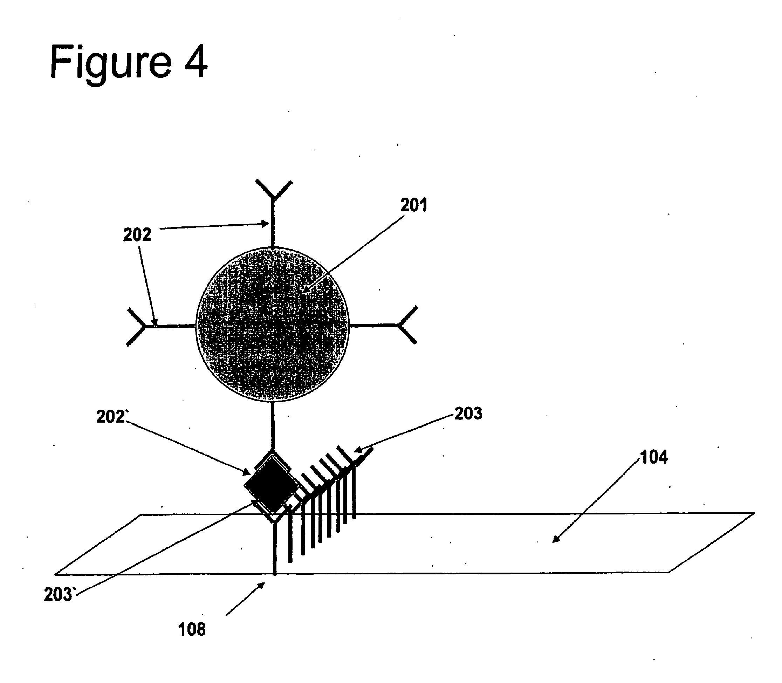

The second specific antibody or antigen is immobilized within the test zone. The complex "target-first colloidal gold conjugate" will be captured by this second antibody or antigen and therefore kept within the test zone to form the sandwich detection (Figures 3 and 4). Then, the second gold conjugate releasing pad will release its gold conjugated with the second specific antibody or antigen to capture the target analyte from the second site. The second conjugate would bind with the first conjugate from the side of the target (Figures 3 and 4). At the same time, the other free sides of the target will be able to link with their specific antibody or antigen to form more and more branched bonds that propagate the accumulation of colloidal gold particles onto the capturing/sample line. This propagation and accumulation of colloidal gold signal will amplify the signal and highly increase the sensitivity. This will enable us to detect very low concentrations that are not detectable using the same technique without signal amplification.

In one embodiment of the device according to the present invention the membrane is attached by means of an adhesive to a supporting backing. Preferably an acrylic pressure sensitive adhesive as known in the art is used.

In another embodiment of the device according to the present invention the first and second gold conjugate pad are laminated between the sample pad and the membrane, wherein the two gold conjugates are separated by a divider.

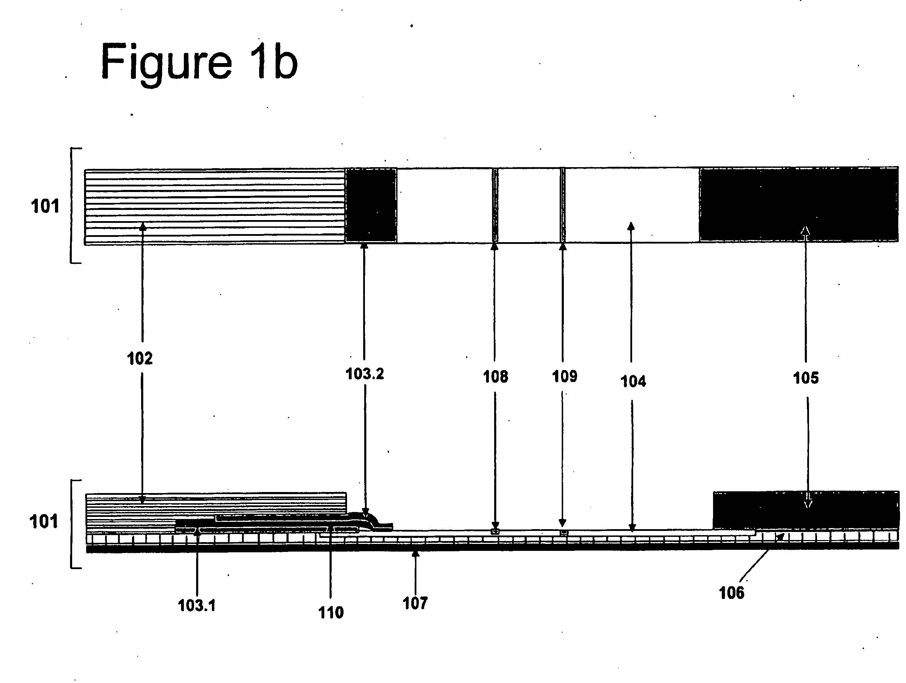

In a preferred embodiment of the device according to the present invention the first 103.1 and second gold conjugate pad 103.2 are laminated between the sample pad 102 and the membrane 104, wherein the two gold conjugates are separated by a divider 110 (Figure 1b). Preferably, the divider is an inert divider, more preferably the divider is a plastic divider

In another embodiment, the device according to the present invention the first gold conjugate pad is attached between the sample pad and the membrane while the second gold conjugate pad is within the upper part of the plastic housing to be released after sample application onto the nitrocellulose membrane directly.

In one preferred embodiment of the device according to the present invention the supporting backing is a plastic backing.

In another preferred embodiment of the device according to the present invention the membrane is nitrocellulose membrane.

In one embodiment of the device according to the present invention the first or second antibody is selected from the group comprising mouse anti-HIV p24, mouse anti-HBsAg, anti-hlgG, anti-Lipoarabinomannan, anti-H.pylori antigen, anti-Leishmania antigen, anti-Pneumonia antigen, anti-Malaria antigen, anti-Chlamydia antigen, anti-Toxoplasma antigen, anti-Schistosoma antigen , HIV 1 antibody, and HIV 2 antibody.

In a preferred embodiment of the device according to the present the first or second antibody is a monoclonal or polyclonal antibody. Preferably the first and second antibodies are two different monoclonal antibodies that recognize the target from two different sites.

In another embodiment of the device according to the present invention the first antigen is selected from the group comprising conjugate of HIV antigen, conjugate of hepatitis C antigen, HIV 1 antigen, HIV 2 antigen, Lipoarabinomannan, H.pylori antigen, Toxoplasma antigen.

In a further embodiment of the device according to the present invention the control zone comprises a non-specific capturing antibody and/or a non-specific antibody capturing protein.

In one preferred embodiment of the device according to the present invention the non-specific antibody is selected from the group consisting of anti-mouse IgG, anti-rabbit IgG, anti-goat IgG, anti-donkey IgG, Anti-sheep IgG, anti-HIV p24, anti-Lipoarabinomannan, anti-H.pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

In another preferred embodiment of the device according to the present invention the non-specific capturing protein is either Protein A or Protein G.

In a preferred embodiment of the device according to the present invention the device comprises a housing comprising at least one test strip according to the present invention.

In another preferred embodiment of the device according to the present invention the housing comprises two, three, four, five, six, seven, eight, nine, or ten test strips. Preferably the housing comprises two, three, four, or five test strips, more preferably the housing comprises two or three test strips.

In one preferred embodiment of the device according to the present invention each test strip contains at least two antibodies or antigens, or at least one antibody and one antigen, wherein one of these antibodies or antigens is immobilized onto the membrane and the other one is conjugated with the first colloidal gold. In case of two antibodies, they have to be different to capture the target from two different sites.

In another aspect the present invention concerns a method for the production of a device according to the present invention, comprising the steps of
a. preparing a colloidal gold solution;
b. preparing a conjugation buffer;
c. partitioning the conjugation buffer by dividing it into a first and a second flask;
d. adding a first specific antibody according to claim 9 to the conjugation buffer in the first flask;
e. adding a second specific antibody according to claim 9 to the conjugation buffer in the second flask, wherein said antibody differs from the antibody used in step d) in that the two antibodies capture the target from two different sites;
f. adding colloidal gold solution into each flask;
g. adding stabilizing buffer to each flask;
h. concentrating each conjugate;
i. adding a surfactant to the first conjugate and soaking glass fibre sheet conjugate pad into the conjugate;
j. soaking another glass fibre sheet conjugate pad into the second conjugate;
k. printing capturing/sample and control lines onto the membrane (104), wherein the sample line comprises the second specific antibody;
l. laminating cards using the glass fibre sheet conjugate pad comprising the first gold conjugate; and
m. cutting cards into strips.

In another aspect the present invention relates to the use of a device according to the present invention for the detection of a disease in at least one sample.

In one preferred embodiment of the use according to the present invention the antibody in one sample (e.g. specimen) and the antigen in another sample (e.g. specimen) is detected. For example, in the case two test strips are used, Lipoarabinomannan-antigen can be detected in urine, while anti-lipoarabinomannan is detected in serum.

In another preferred embodiment of the use according to the present invention the antibody and antigen are detected in the same sample (specimen). For example, HIV antibodies and the HIV p24 antigen are detected in the same serum sample (specimen) using a device of two different strips.

In one embodiment of the use of the device according to the present invention the sample was obtained from a human.

In one preferred embodiment of the use of the device according to the present invention the sample is selected from the group comprising of whole blood, serum, plasma, saliva, and urine.

In another preferred embodiment of the use of the device according to the present invention the disease detected in said sample is selected from the group consisting of HIV, Hepatitis A, Hepatitis B, Hepatitis C, H.pylori, Leishmania, Schistosomiasis, Malaria, Pneumonia, Toxoplasmosis, Tubercolosis and Chlamydial infection.

In a further aspect the present invention refers to a kit for detection of a disease comprising the device according to the present invention and a manual.

In one preferred embodiment of the kit according to the present invention the kit further comprises an assay buffer. The assay buffer can be any buffer known in the art suitable for the use of whole blood samples. Preferably in the case of whole blood samples Tris buffer is used, more preferably 0.1M Tris buffer having a pH of 7.5 and comprising a preservative. Any preservative known by a person skilled in the art can be used, preferably sodium azide and even more preferably 0.01M sodium azide is used.

In another embodiment the present invention relates to the use of the method for diagnosing and monitoring a disease or a specific condition of a subject by detecting a target in a sample.

The following example illustrate the present invention without, however, limiting the same thereto.

### BRIEF DESCRIPTION OF THE DRAWING

**Figure 1a****:** shows top and side views of a typical rapid-flow immunochromatographic test device known in the art in the form of a test strip 101 comprising a sample pad 102, a conjugate pad 103, a membrane 104, an absorbent pad 105, an adhesive 106, a supporting backing 107, a test zone 108, and a control zone 109.
**Figure 1b****:** shows top and side views of a preferred embodiment of a rapid-flow immunochromatographic test device according to the present invention in the form of a test strip 101 comprising a sample pad 102, a first conjugate pad 103.1, a second conjugate pad 103.2, a membrane 104, an absorbent pad 105, an adhesive 106, a supporting backing 107, a test zone 108, a control zone 109, and the conjugates divider 110.

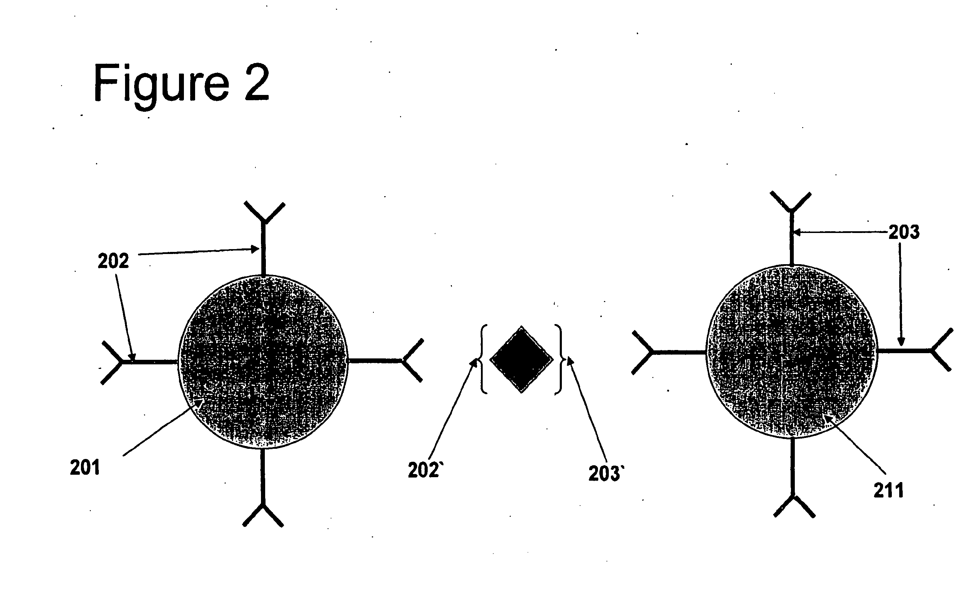

**Figure 2****:** shows the schematically view of a preferred embodiment of the first and second colloidal gold according to the present invention, wherein the first colloidal gold 201 is conjugated with a first specific antibody 202 and wherein the second colloidal gold 211 is conjugated with a second specific antibody 203. In addition, the target is shown having two sides 202' and 203'. The first side 202' of the target is captured by the first antibody 202 of the first gold conjugate 201 and the second side 203' of the target is captured by the second antibody 203 conjugated with the second gold conjugate 211.
**Figure 3****:** shows a simplified scheme of a preferred embodiment of the test device according to the present invention. It shows the test zone 108 of the membrane 104 on the test strip 101, wherein the second specific antibody 203 or antigen is immobilized to the test zone 108.
**Figure 4****:** shows the main principle of a preferred embodiment of the signal development according to the present invention. By the sample flow within the rapid immunochromatographic test device the target in the sample will be captured by the first specific antibody 202 or antigen of the first colloidal gold 201 to form the complex "target-first colloidal gold". This complex flows to the test zone 108, where it will be captured by the second specific antibody 203 or antigen that is immobilized onto the membrane 104 to form a sandwich detection.

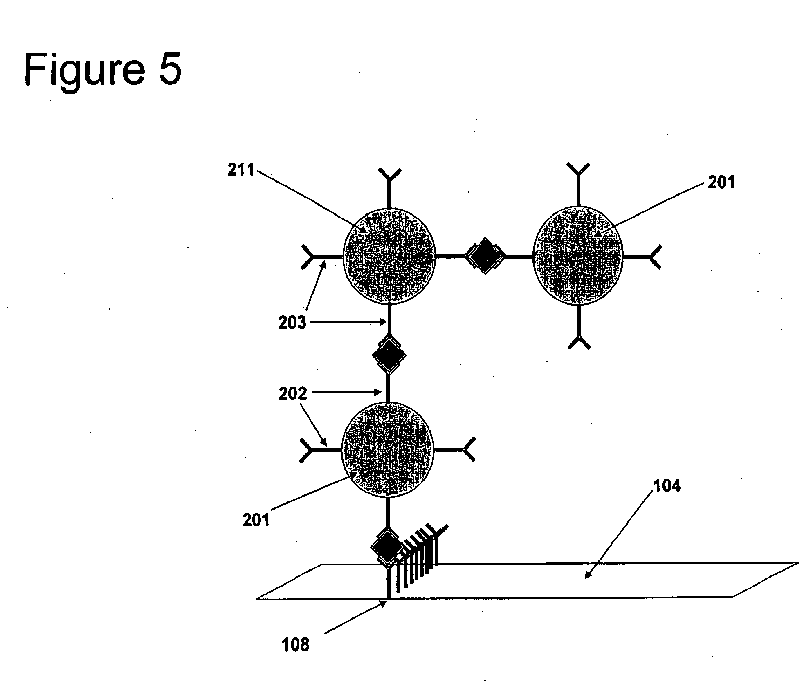

**Figure 5****:** shows the main principle of a preferred embodiment of the signal amplification and multiplication according to the present invention. By the sample flow within the rapid immunochromatographic test device the target in the sample will be captured by the first specific antibody 202 or antigen that is conjugated to the first colloidal gold 201 to form the complex "target- first colloidal gold". This complex flows to the test zone 108, where it will be captured by the second specific antibody 203 or antigen that is immobilized onto the membrane 104 of the test zone 108. Then, the second colloidal gold 211 conjugated with the second specific antibody 203 or antigen will be released and will bind to the target as well as to the first colloidal gold conjugate 201 and enhance the signal by forming a double sandwich.

### EXAMPLES

The following examples illustrate the present invention without, however, limiting the same thereto.

### Example 1: Preparation of an preferred embodiment of a test device according to the present invention

a) prepare 1% aqueous solution of tetrachloroauric acid at room temperature;
b) prepare 4% trisodium citrate aqueous solution at room temperature;
c) prepare 0.05 M Potassium Carbonate aqueous solution at room temperature;
d) prepare 600ml of phosphate stabilizing buffer of pH 7.4, containing BSA, Tween 20, Sucrose, polyvinylpurrolidone and a preservative, e.g. sodium azide, at room temperature;
e) prepare colloidal gold solution by reduction of 1.7 ml boiling tetrachloroauric acid solution (after dilution into 100ml) using 1ml trisodium citrate solution and let it takes the room temperature;
f) dilute the colloidal gold solution as 1:1 using distilled water. Adjust the pH to 7.4 using potassium carbonate solution at room temperature;
g) prepare 200ml of phosphate conjugation buffer of pH 7.4 at room temperature;
h) partition the 200ml conjugation buffer by dividing it into two flasks (100ml of each);
i) add 1.0 mg of aqueous antibody (e.g. anti-p24 1^{st} clone) to the conjugation buffer in the first flask with stirring at room temperature;
j) add 1.0 mg of aqueous antibody (e.g. anti-p24 2^{nd} clone) to the conjugation buffer in the second flask with stirring at room temperature;
k) add 100ml colloidal gold solution into each flask with stirring at room temperature;
l) after about 45 minutes; add 200ml of stabilizing buffer to each flask;
m) after about 20 minutes; concentrate each conjugate by cooled (temperature around 15°C ) high speed centrifugation (10,000 rpm for one hour);
n) discard the supernatant and re-suspend the concentrated conjugates using the stabilising buffer at room temperature;
o) adjust the concentration for each of the two conjugates to O.D.₅₂₀=2.0;
p) add 0.1ml of Tween 20 to the first conjugate and soak glass fibre sheet conjugate pad into the conjugate, then heat dry at temperature around 50°C; and
q) soak another glass fiber sheet conjugate pad into the second conjugate, then heat dry at temperature around 50°C.
(* In case of antibodies/antigens and their specific antigens/antibodies there is no need for these steps of bovine serum albumin or any other protein labelling. ** Other proteins or peptides could be used other than bovine serum albumin).

Additionally, print sample (e.g. anti-p24, 2^{nd} clone) and control lines (e.g. anti-mouse IgG) onto nitrocellulose membrane, then heat dry at temperature around 50°C.

Finally, laminate cards according to the following procedure:

### A. In case of conjugate releasing site laminated within the upper side of the device plastic housing

Lamination of cards using the first gold conjugate. Laminate card components onto the backing material with the sequence:
1. laminate the nitrocellulose membrane nearly in the middle of the card;
2. laminate the absorbent pad in the end of the card (overlaps from the nitrocellulose membrane side);
3. laminate the first conjugate pad in the other side of the nitrocellulose membrane; and
4. laminate the sample pad.

### B. In case of conjugate releasing site laminated onto the test strip itself separated from the first conjugate by a divider

Laminate card components onto the backing material with the sequence (see figure 1b):
1. laminate the nitrocellulose membrane nearly in the middle of the card;
2. laminate the absorbent pad in the end of the card (overlaps from the nitrocellulose membrane side);
3. laminate the first conjugate pad in the other side of the nitrocellulose membrane ;
4. laminate the plastic divider onto the first conjugate (overlaps from the nitrocellulose membrane side);
5. laminate the second conjugate pad onto the divider (overlaps from the nitrocellulose membrane side);
6. laminate the sample pad onto the other end of the card, the sample pad will overlaps with the two conjugate pads; and
7. then cut cards into strips.

### C. Alternatively

Lamination of the second gold conjugate could be applied within the plastic housing itself to ensure that the two conjugates will not propagate before release from the releasing pad and so stick within the releasing pad.

### Example 2: Hepatitis B surface antigen (HBsAg) detection system

The first gold conjugate pad contains a conjugate of colloidal gold with a first mouse monoclonal anti-HBsAg , and the second gold conjugate pad contains a conjugate of colloidal gold with a second mouse monoclonal anti-HBsAg. The first conjugate releasing pad 103.1 is laminated on the test strip between the sample pad and the nitrocellulose membrane 104 while the second 103.2 is above the first pad separated by a divider 110 to be released directly toward the nitrocellulose membrane 104 without flow through the first conjugate pad to avoid interact with the first conjugate before reaching the membrane (Figure 1b).
The second conjugate releasing site could be laminated within the upper side of the device plastic housing.

The sample line 108 is the second mouse monoclonal anti-HBsAg immobilized onto the nitrocellulose membrane 104. The control line 109 is anti-mouse IgG. Sample 108 and control lines 109 turn into purple color in case of HBsAg availability in the sample; only the control line 109 turns into purple color in case of HBsAg free sample, see Figure1b.

The commercially available rapid tests sensitivity for Hepatitis B surface antigen is within the range 500-1000µg/ml while according to this system it is so simple to detect less than 200 pg/ml.

### Example 3: Human Immunodeficiency Virus (HIV) antibodies detection system

The first gold conjugate pad contains a conjugate of colloidal gold with a first mouse antihuman Immunoglobulin G (anti-hIgG), and the second gold conjugate pad contains a conjugate of colloidal gold with HIV p160. The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane (Figure 1b).
The second conjugate releasing site could be laminated within the upper side of the device plastic housing.

The sample line 108 is HIV p160 antigen immobilized onto the nitrocellulose membrane 104. The control line 109 is anti-mouse IgG. Sample 108 and control lines 109 turn into purple colour in case of HIV antibodies availability in the sample; only the control line 109 turns into purple colour in case of HIV antibodies free sample, see Figure 1b.

According to this system it is so simple to detect very low titers of HIV antibodies.

### References

(1) J Chandler, N Robinson, and K Whiting, "Handling False Signals in Gold-Based Rapid Tests", IVD Technology 7, no. 2 (2001): 34-45; http://www.devicelink.com/ivdt/archive/01/03/002.html.
(2) J Chandler, T Gurmin, and N Robinson, "The Place of Gold in Rapid Tests", IVD Technology 6, no. 2 (2000): 37-49; http://www.devicelink.com/ivdt/archive/00/03/004.html
(3) TC Tisone et al., "Image Analysis for Rapid-Flow Diagnostics", IVD Technology 5, no. 5 (1999): 52-58; http://www.devicelink.com/ivdt/archive/99/09/010.html.
(4) Zaaijer, H.L., Exel-Oehlers, P.V., Kraaijeveld, T., Altena, E., Lelie, P.N. (1992) Early detection of antibodies to HIV-1 by third-generation assays. Lancet 340, 770-772.
(5) Constantine, N.T., van der Groen, G., Belsey, E.M., Tamashiro, H. (1994) Sensitivity of HIV-antibody assays determined by seroconversion panels. AIDS 8, 1715-1720.
(6) Satten, G.A., Busch, M.P., et al. (1997) Effect of transmission route on window period estimates. Fourth Conference on Retroviruses and Opportunistic Infections, Washington DC, Abstract 122.
(7) WHO. Rapid HIV tests: Guidelines for use in HIV testing and counseling services in resource-constrained settings. Geneva 2004. http://www.who.int/hiv/pub/vct/rapidhivtests/en/
(8) Holodniy M, et al. (1991) Reduction in plasma human immunodeficiency virus ribonucleic acid following dideoxynucleoside therapy as determined by the polymerase chain reaction. J. Clin. Invest 88, 1755-1759.
(9) Katzenstein D.A., et al. (1994) Quantitation of human immunodeficiency virus by culture and polymerase chain reaction in response to didanosine after long-term therapy with zidovudine. J. Infect. Dis. 169, 416-419.
(10) Jackson JB, et al. (1998) Practical diagnostic testing for human immunodeficiency virus. Clin. Microb. Rev. 1, 124-138.
(11) Goudsmit J, et al. (1986) Expression of human Immunodeficiency virus antigen (HIV-Ag) in serum and cerebrospinal fluid during acute and chronic infection. Lancet 2, 177-180.
(12) Aubuchon, J.P., Birkmeyer, J.D., Busch, M.P. (1997) Cost-effectiveness of expanded human immunodeficiency virus-testing protocols for donated blood. Transfusion 45, 45-51.
(13) Ward, J.M., Holmberg, S.D., Allen, J.R., Cohn, D.L., et al. (1988) Transmission of human immunodeficiency virus (HIV) by blood transfusion screened as negative for HIV antibody. N. Engl. J. Med. 8, 473-478.
(14) Alter, H.J., et al. (1990) Prevalence of human immunodeficiency virus type 1 p24 antigen in U.S. blood donors - an assessment of the efficacy of testing in donor screening. N. Engl. J. Med. 323, 1312-1317.
(15) Mayers, D.L. (1998) Drug-resistant HIV-1. The virus strikes back. JAMA 279, 2000-2002.
(16) Stephenson, J. (2002) Cheaper HIV drugs for poor nations bring a new challenge: monitoring treatment. JAMA 288, 2.
(17) WHO. HIV assays: Operational characteristics (Phase 1). Report 15/ antigen/antibody ELISAs. Geneva 2004. http://www.who.int/diagnostics_laboratory_evaluations/hiv/en/
(18) WHO. HIV assays: Operational characteristics (Phase 1). Report 14/ simple/rapid tests. Geneva 2003. http://www.who.int/diagnostics_laboratory/evaluations/hiv/en/
(19) Meier, T, et al. (2001) Evidence for a diagnostic window in fourth generation assays for HIV. J. Clin. Virol. 23, 113-116.
(20) Wang, X. and Zhan, W. (2006) Development of an immunochromatographic test to detect White Spot Syndrome Virus of shrimp. Aquaculture 255, 196-200.
(21) Oh, J. et al. (1999) Evaluation of Genedia® HBsAg Rapid and Genedia® Anti-HBs Rapid for the screening of HBsAg and anti-HBs. Korean J. Clin. Pathol. 19, 114-117.

## Claims

1. A rapid immunochromatographic test device for the detection of a target in a sample, comprising
a) a first gold conjugate releasing pad, comprising colloidal gold conjugated with a first specific antibody or specific antigen to capture the target from a first site,
(b) a second gold conjugate releasing pad, comprising colloidal gold conjugated with a second specific antibody or specific antigen to capture the target from a second site; and
(c) a capture test zone comprising the second specific antibody or specific antigen; wherein both releasing pads are located at different positions within the test device such that the target in the sample will be captured by the first specific antibody or specific antigen conjugated to the first colloidal gold to form a complex "target-first colloidal gold conjugate", the complex will be captured then by the second specific antibody or specific antigen and kept within the test zone, and then the second colloidal gold conjugated with the second specific antibody or specific antigen will be released to capture the target.

2. The device according to claim 1, wherein said device comprises a test strip comprising
(a) a sample pad;
(b) a conjugate pad comprising said first gold conjugate releasing pad;
(c) a conjugate pad comprising said second gold conjugate releasing pad;
(d) a membrane comprising the capture test zone and a negative control zone; and
(e) an absorbent pad.

3. The device according to claim 2, wherein said capture test zone comprises said second specific antibody or specific antigen.

4. The device according to claim 2 or 3, wherein said membrane is attached by means of an adhesive to a supporting backing.

5. The device according to any of claims 2-4, wherein said first and second gold conjugate pad are laminated between the sample pad and the membrane, wherein said two gold conjugate pads are separated by a divider.

6. The device according to any of claims 2-4, said first gold conjugate pad is attached between the sample pad and the membrane while the second gold conjugate pad is within the upper part of the plastic housing.

7. The device according to claims 4-6, wherein said supporting backing is a plastic backing.

8. The device according to any of claims 2-7, wherein said membrane is nitrocellulose membrane.

9. The device according to any of claims 1-8, wherein said first specific antibody or second specific antibody is selected from the group comprising mouse anti-HIV p24, mouse anti-HBsAg, anti-hlgG, anti-Lipoarabinomannan, anti-H.pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

10. The device according to any of claims 1-8, wherein said first specific antigen is selected from the group comprising conjugate of HIV antigen, conjugate of hepatitis C antigen, HIV 1 antigen (HIV p160), HIV 2 antigen (HIV p36), Hepatitis B antigen, Lipoarabinomannan, H. pylori antigen, Toxoplasma antigen.

11. The device according to any of claims 2-10, wherein said control zone comprises a non-specific capturing antibody and/or a non-specific capturing protein.

12. The device according to claim 11, wherein said non-specific antibody is selected from the group consisting of anti-mouse IgG, anti-rabbit IgG, anti-goat IgG, anti-donkey IgG, Anti-sheep IgG, anti-HIV p24, anti-Lipoarabinomannan, anti-H. pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

13. The device according to claim 11, wherein said non-specific capturing protein selected is either Protein A or Protein G.

14. A method for the production of a device according to any of claims 1-13, comprising the steps of
a. preparing a colloidal gold solution;
b. preparing a conjugation buffer;
c. partitioning the conjugation buffer by dividing it into a first and a second flask;
d. adding a first specific antibody according to claim 9 to the conjugation buffer in the first flask;
e. adding a second specific antibody according to claim 9 to the conjugation buffer in the second flask, wherein said antibody differs from the antibody used in step d) in that the two antibodies capture the target from two different sites;
f. adding colloidal gold solution into each flask;
g. adding stabilizing buffer to each flask;
h. concentrating each conjugate;
i. adding a surfactant to the first conjugate and soaking glass fibre sheet conjugate pad into the conjugate;
j. soaking another glass fibre sheet conjugate pad into the second conjugate;
k. printing capturing/sample and control lines onto the membrane (104), wherein the sample line comprises the second specific antibody;
l. laminating cards using the glass fibre sheet conjugate pad comprising the first gold conjugate; and
m. cutting cards into strips.

15. Use of a device according to any of claims 1-13 for the detection of a disease in at least one sample.

16. The use according to claim 15, wherein said sample was obtained from a human.

17. The use according to claim 16, wherein said sample is selected from the group comprising of whole blood, serum, plasma, saliva, and urine.

18. The use according to any of claims 15-17, wherein said disease detected in said sample is selected from the group consisting of HIV, Hepatitis A. Hepatitis B, Hepatitis C, H. pylori, Leishmania, Schistosomiasis, Malaria, Pneumonia, Toxoplasmosis, culosis and Chlamydia infection.

19. Kit for detection of a disease comprising the device according to any of claims 1-13 and a manual.

20. The kit according to claim 19 further comprising an assay buffer.

21. The kit according to claim 20, wherein said assay buffer comprises a preservative.

## Patentansprüche

1. Immunochromatographische Schnelltestvorrichtung zur Detektion eines Targets in einer Probe, umfassend
(a) ein erstes Goldkonjugatfreisetzungskissen, das kolloidales Gold umfasst, konjugiert mit einem ersten spezifischen Antikörper oder spezifischen Antigen, um das Target von einer ersten Stelle einzufangen,
(b) ein zweites Goldkonjugatfreisetzungskissen, das kolloidales Gold umfasst, konjugiert mit einem zweiten spezifischen Antikörper oder spezifischen Antigen, um das Target von einer zweiten Stelle einzufangen; und
(c) eine Einfangtestzone, die den zweiten spezifischen Antikörper oder das zweite spezifische Antigen umfasst;
wobei beide Freisetzungskissen an unterschiedlichen Positionen innerhalb der Testvorrichtung angeordnet sind, so dass das Target in der Probe durch den ersten spezifischen Antikörper oder durch das erste spezifische Antigen, der/das an das erste kolloidale Gold konjugiert ist, eingefangen werden wird, um einen Komplex "Targeterstes kolloidales Goldkonjugat" zu bilden, der Komplex dann durch den zweiten spezifischen Antikörper oder durch das zweite spezifische Antigen eingefangen und innerhalb der Testzone gehalten werden wird und dann das zweite kolloidale Gold, das mit dem zweiten spezifischen Antikörper oder spezifischen Antigen konjugiert ist, freigesetzt werden wird, um das Target einzufangen.

2. Vorrichtung nach Anspruch 1, wobei die Vorrichtung einen Teststreifen umfasst, der
(a) ein Probenkissen;
(b) ein Konjugatkissen, das das erste Goldkonjugatfreisetzungskissen umfasst;
(c) ein Konjugatkissen, das das zweite Goldkonjugatfreisetzungskissen umfasst;
(d) eine Membran, die die Einfangtestzone und eine Negativkontrollzone umfasst; und
(e) ein Absorptionskissen
umfasst.

3. Vorrichtung nach Anspruch 2, wobei die Einfangtestzone den zweiten spezifischen Antikörper oder das zweite spezifische Antigen umfasst.

4. Vorrichtung nach Anspruch 2 oder 3, wobei die Membran mittels eines Klebers an einer Tragerückschicht befestigt ist.

5. Vorrichtung nach einem der Ansprüche 2-4, wobei das erste und zweite Goldkonjugatkissen zwischen das Probenkissen und die Membran laminiert sind, wobei die zwei Goldkonjugatkissen durch einen Teiler getrennt sind.

6. Vorrichtung nach einem der Ansprüche 2-4, wobei das erste Goldkonjugatkissen zwischen dem Probenkissen und der Membran angebracht ist, während das zweite Goldkonjugatkissen sich im oberen Teil des Kunststoffgehäuses befindet.

7. Vorrichtung nach den Ansprüchen 4-6, wobei die Tragerückschicht eine Kunststoffrückschicht ist.

8. Vorrichtung nach einem der Ansprüche 2-7, wobei die Membran Nitrocellulose-Membran ist.

9. Vorrichtung nach einem der Ansprüche 1-8, wobei der erste spezifische Antikörper oder zweite spezifische Antikörper ausgewählt ist aus der Gruppe, umfassend Maus-anti-HIV p24, Maus-anti-HBsAg, anti-hlgG, anti-Lipoarabinomannan, anti-H. pylori-Antigen, anti-Leishmania-Antigen, anti-Pneumonia-Antigen, anti-Malaria-Antigen, anti-Chlamydia-Antigen, anti-Toxoplasma-Antigen, anti-Schistosoma-Antigen, HIV-1-Antikörper und HIV-2-Antikörper.

10. Vorrichtung nach einem der Ansprüche 1-8, wobei das erste spezifische Antigen ausgewählt ist aus der Gruppe, umfassend Konjugat von HIV-Antigen, Konjugat von Hepatitis-C-Antigen, HIV-1-Antigen (HIV p160), HIV-2-Antigen (HIV p36), Hepatitis-B-Antigen, Lipoarabinomannan, H. pylori-Antigen, Toxoplasma-Antigen.

11. Vorrichtung nach einem der Ansprüche 2-10, wobei die Kontrollzone einen nichtspezifischen Einfangantikörper und/oder ein nicht-spezifisches Einfangprotein umfasst.

12. Vorrichtung nach Anspruch 11, wobei der nicht-spezifische Antikörper ausgewählt ist aus der Gruppe, bestehend aus anti-Maus-IgG, anti-Kaninchen-IgG, anti-Ziege-IgG, anti-Esel-IgG, anti-Schaf-IgG, anti-HIV p24, anti-Lipoarabinomannan, anti-H. pylori-Antigen, anti-Leishmania-Antigen, anti-Pneumonia-Antigen, anti-Malaria-Antigen, anti-Chlamydia-Antigen, anti-Toxoplasma-Antigen, anti-Schistosoma-Antigen, HIV-1-Antikörper und HIV-2-Antikörper.

13. Vorrichtung nach Anspruch 11, wobei das nicht-spezifische Einfangprotein ausgewählt ist aus entweder Protein A oder Protein G.

14. Verfahren zur Herstellung einer Vorrichtung nach einem der Ansprüche 1-13, das die Schritte umfasst
a. Herstellen einer kolloidalen Goldlösung;
b. Herstellen eines Konjugationspuffers;
c. Aufteilen des Konjugationspuffers durch Verteilen desselben in einen ersten und einen zweiten Kolben;
d. Zugeben eines ersten spezifischen Antikörpers nach Anspruch 9 zum Konjugationspuffer im ersten Kolben;
e. Zugeben eines zweiten spezifischen Antikörpers nach Anspruch 9 zum Konjugationspuffer im zweiten Kolben, wobei der Antikörper sich vom in Schritt d) verwendeten Antikörper darin unterscheidet, dass die zwei Antikörper das Target von zwei unterschiedlichen Stellen einfangen;
f. Zugeben von kolloidaler Goldlösung in jeden Kolben;
g. Zugeben von Stabilisierungspuffer zu jedem Kolben;
h. Konzentrieren jeden Konjugats;
i. Zugeben eines Tensids zum ersten Konjugat und Tränken von Glasfaserschichtkonjugatkissen im Konjugat;
j. Tränken eines weiteren Glasfaserschichtkonjugatkissens im zweiten Konjugat;
k. Drucken von Einfang/Proben- und Kontrolllinien auf die Membran (104), wobei die Probenlinie den zweiten spezifischen Antikörper umfasst;
l. Laminieren von Karten unter Verwendung des Glasfaserschichtkonjugatkissens, das das erste Goldkonjugat umfasst; und
m. Zuschneiden der Karten zu Streifen.

15. Verwendung einer Vorrichtung nach einem der Ansprüche 1-13 zur Detektion einer Erkrankung in wenigstens einer Probe.

16. Verwendung nach Anspruch 15, wobei die Probe von einem Menschen erhalten wurde.

17. Verwendung nach Anspruch 16, wobei die Probe ausgewählt ist aus der Gruppe, die Vollblut, Serum, Plasma, Speichel und Harn umfasst.

18. Verwendung nach einem der Ansprüche 15-17, wobei die Erkrankung, die in der Probe nachgewiesen wird, ausgewählt ist aus der Gruppe, bestehend aus HIV, Hepatitis A, Hepatitis B, Hepatitis C, H. pylori, Leishmania, Schistosomiasis, Malaria, Pneumonie, Toxoplasmose, Tuberkulose und Chlamydia-Infektion.

19. Kit zum Nachweis einer Erkrankung, der die Vorrichtung nach einem der Ansprüche 1-13 und eine Anleitung umfasst.

20. Kit nach Anspruch 19, der weiter einen Testpuffer umfasst.

21. Kit nach Anspruch 20, wobei der Testpuffer einen Konservierungsstoff umfasst.

## Revendications

1. Dispositif d'essai rapide immunochromatographique pour la détection d'une cible dans un échantillon, comprenant
(a) un premier tampon de libération de conjugué constitué d'or, comprenant de l'or colloïdal conjugué à un premier anticorps spécifique ou antigène spécifique pour capturer la cible à partir d'un premier site,
(b) un deuxième tampon de libération de conjugué constitué d'or, comprenant de l'or colloïdal conjugué à un deuxième anticorps spécifique ou antigène spécifique pour capturer la cible à partir d'un deuxième site ; et
(c) une zone d'essai de capture comprenant le deuxième anticorps spécifique ou antigène spécifique ;
dans lequel les deux tampons de libération sont situés à différentes positions dans le dispositif d'essai de sorte que la cible dans l'échantillon sera capturée par le premier anticorps spécifique ou antigène spécifique conjugué au premier or colloïdal pour former un complexe "cible-premier conjugué constitué d'or colloïdal", le complexe sera capturé ensuite par le deuxième anticorps spécifique ou antigène spécifique et maintenu dans la zone d'essai, puis le deuxième or colloïdal conjugué au deuxième anticorps spécifique ou antigène spécifique sera libéré pour capturer la cible.

2. Dispositif selon la revendication 1, dans lequel ledit dispositif comprend une bande d'essai comprenant
(a) un tampon à échantillon ;
(b) un tampon conjugué comprenant ledit premier tampon de libération de conjugué constitué d'or ;
(c) un tampon conjugué comprenant ledit deuxième tampon de libération de conjugué constitué d'or ;
(d) une membrane comprenant la zone d'essai de capture et une zone de contrôle négatif ; et
(e) un tampon absorbant.

3. Dispositif selon la revendication 2, dans lequel ladite zone d'essai de capture comprend ledit deuxième anticorps spécifique ou antigène spécifique.

4. Dispositif selon la revendication 2 ou 3, dans lequel ladite membrane est attachée au moyen d'un adhésif à un support.

5. Dispositif selon l'une des revendications 2-4, dans lequel lesdits premier et deuxième tampons de conjugués constitués d'or sont stratifiés entre le tampon à échantillon et la membrane, dans lequel lesdits deux tampons de conjugués constitués d'or sont séparés par un séparateur.

6. Dispositif selon l'une des revendications 2-4, dans lequel ledit premier tampon de conjugué constitué d'or est attaché entre le tampon à échantillon et la membrane alors que le deuxième tampon de conjugué constitué d'or se trouve dans la partie supérieure du coffret plastique.

7. Dispositif selon les revendications 4-6, dans lequel ledit support est un support plastique.

8. Dispositif selon l'une des revendications 2-7, dans lequel ladite membrane est une membrane de nitrocellulose.

9. Dispositif selon l'une des revendications 1-8, dans lequel ledit premier anticorps spécifique ou deuxième anticorps spécifique est sélectionné dans le groupe composé d'un anticorps de souris anti-protéine p24 du VIH, d'un anticorps de souris anti-HBsAg, d'un anticorps anti-hlgG, d'un anticorps anti-Lipoarabinomannan, d'un anticorps dirigé contre l'antigène anti-H. pylori, d'un anticorps dirigé contre l'antigène de Leishmania, d'un anticorps dirigé contre l'antigène de Pneumonia, d'un anticorps dirigé contre l'antigène de la Malaria, d'un anticorps dirigé contre l'antigène de Chlamydia, d'un anticorps dirigé contre l'antigène de Toxoplasma, d'un anticorps dirigé contre l'antigène de Schistosoma, d'un anticorps anti-VIH-1, et d'un anticorps anti-VIH-2.

10. Dispositif selon l'une des revendications 1-8, dans lequel ledit premier antigène spécifique est sélectionné dans le groupe composé d'un conjugué d'un antigène du VIH, d'un conjugué d'un antigène de l'hépatite C, d'un antigène du VIH-1 (antigène p160 du VIH), d'un antigène du VIH-2 (antigène p36 du VIH), d'un antigène de l'Hépatite B, d'un antigène de Lipoarabinomannan, d'un antigène anti-H. pylori, d'un antigène de Toxoplasma.

11. Dispositif selon l'une des revendications 2-10, dans lequel ladite zone de contrôle comprend un anticorps de capture non-spécifique et/ou une protéine de capture non-spécifique.

12. Dispositif selon la revendication 11, dans lequel ledit anticorps non spécifique est sélectionné dans le groupe composé d'un anticorps anti-IgG de souris, d'un anticorps anti-IgG de lapin, d'un anticorps anti-IgG de chèvre, d'un anticorps anti-IgG de l'âne, d'un anticorps anti-IgG de mouton, d'un anticorps anti protéine p24 du VIH, d'un anticorps anti-Lipoarabinomannan, d'un anticorps dirigé contre l'antigène anti-H. pylori, d'un anticorps dirigé contre l'antigène de Leishmania, d'un anticorps dirigé contre l'antigène de Pneumonia, d'un anticorps dirigé contre l'antigène de la Malaria, d'un anticorps dirigé contre l'antigène de Chlamydia, d'un anticorps dirigé contre l'antigène de Toxoplasma, d'un anticorps dirigé contre l'antigène de Schistosoma, d'un anticorps anti-VIH-1, et d'un anticorps anti-VIH-2.

13. Dispositif selon la revendication 11, dans lequel ladite protéine de capture non spécifique sélectionnée est soit la protéine A soit la protéine G.

14. Procédé pour la fabrication d'un dispositif selon l'une des revendications 1-13, comprenant les étapes qui consistent à
a. préparer une solution constituée d'or colloïdal ;
b. préparer un tampon de conjugué ;
c. répartir le tampon de conjugué en le divisant en un premier et un deuxième flacon ;
d. ajouter un premier anticorps spécifique selon la revendication 9 dans le tampon de conjugué dans le premier flacon ;
e. ajouter un deuxième anticorps spécifique selon la revendication 9 dans le tampon de conjugué dans le deuxième flacon, où ledit anticorps diffère de l'anticorps utilisé dans l'étape d) en ce que les deux anticorps capturent la cible à partir de deux sites différents ;
f. ajouter une solution constituée d'or colloïdal dans chaque flacon ;
g. ajouter le tampon de stabilisation à chaque flacon ;
h. concentrer chaque conjugué ;
i. ajouter un tensioactif au premier conjugué et tremper un tampon de conjugué en feuille en fibres de verre dans le conjugué ;
j. tremper un autre tampon de conjugué en feuille en fibres de verre dans le deuxième conjugué ;
k. imprimer/capturer des lignes de contrôle et d'échantillon/de capture sur la membrane (104), où la ligne d'échantillon comprend le deuxième anticorps spécifique ;
l. stratifier des cartes en utilisant le tampon de conjugué en feuille en fibres de verre comprenant le premier conjugué constitué d'or ; et
m. couper les cartes en bandes.

15. Utilisation d'un dispositif selon l'une des revendications 1-13 pour la détection d'une maladie dans au moins un échantillon.

16. Utilisation selon la revendication 15, dans laquelle ledit échantillon a été obtenu d'un humain.

17. Utilisation selon la revendication 16, dans laquelle ledit échantillon est sélectionné dans le groupe comprenant du sang total, du sérum, du plasma, de la salive et de l'urine.

18. Utilisation selon l'une des revendications 15-17, dans laquelle ladite maladie détectée dans ledit échantillon est sélectionnée dans le groupe composé du VIH, de l'hépatite A, de l'hépatite B, de l'hépatite C, d'une infection H. pylori, de la Leishmania, de la Schistosomiasis, de la malaria, de la pneumonie, de la toxoplasmose, de la tuberculose et de l'infection de Chlamydia.

19. Trousse pour la détection d'une maladie comportant le dispositif selon l'une des revendications 1-13 et un manuel.

20. Trousse selon la revendication 19 comprenant en outre un tampon de dosage.

21. Trousse selon la revendication 20, dans lequel ledit tampon de dosage comprend un agent de conservation.
